(19) Europäisches Patentamt
European Patent Office
Office européen des brevets

(11) Veröffentlichungsnummer: **0 066 769**
**B1**

(12) # EUROPÄISCHE PATENTSCHRIFT

(45) Veröffentlichungstag der Patentschrift: **31.07.85**

(51) Int. Cl.⁴: **A 01 N 31/04**, B 27 K 3/40 // C07C33/48

(21) Anmeldenummer: **82104533.3**

(22) Anmeldetag: **25.05.82**

(54) **Fungizide Mittel für den Materialschutz.**

(30) Priorität: **04.06.81 DE 3122263**

(43) Veröffentlichungstag der Anmeldung: **15.12.82 Patentblatt 82/50**

(45) Bekanntmachung des Hinweises auf die Patenterteilung: **31.07.85 Patentblatt 85/31**

(84) Benannte Vertragsstaaten: **AT CH DE FR GB LI SE**

(56) Entgegenhaltungen:
**EP - A - 0 002 679**
**EP - A - 0 066 761**
**EP - A - 0 066 771**
**CH - A - 601 151**
**FR - A - 913 365**
**FR - A - 1 068 853**
**FR - A - 1 404 792**
**FR - A - 1 474 706**
**FR - A - 2 070 552**

**CHEMICAL ABSTRACTS, Band 85, Nr. 21, 22. November 1976, Seite 492, Nr. 159417t, Columbus Ohio (USA);**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Jäger, Gerhard, Dr., Gellertstrasse 18, D-5090 Leverkusen (DE)**
Erfinder: **Paulus, Wilfried, Dr., Deswatinesstrasse 90, D-4150 Krefeld (DE)**
Erfinder: **Genth, Hermann, Dr., Am Heckerhof 60, D-4150 Krefeld (DE)**

**EP 0 066 769 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft die Verwendung von 1-Iod-1-alkinolen als fungizide Mittel zum Schutz technischer Materialien.

Aus der EP-A 0 002 679 sind 1-Halogen-1-propen-3-ole und ihre Verwendung zur Bekämpfung von Phytophtora infestans sowie von Venturia-Arten bekannt. Außerdem können sie zur Bekämpfung von Getreidekrankheiten, wie Getreiderost und Weizensteinbrand, eingesetzt werden.

In der FR-A 1 404 792 werden Iod-propargyl-carbinole beschrieben, die das Wachstum von Pilzen und Bakterien hemmen und auch als Desinfektionsmittel verwendet werden können.

Überraschenderweise hat sich gezeigt, daß 1-Iod-1-alkinole der Formel

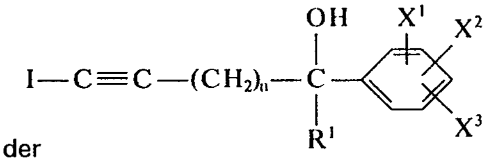

(I)

in der

$X^1$ $X^2$ und $X^3$ gleich oder verschieden sind und Wasserstoff, Fluor, Chlor oder Brom bedeuten,
n für die Zahlen 0 oder 1 steht und
$R^1$ Niederalkyl mit 1 bis etwa 6 Kohlenstoffatomen, Cycloalkyl mit 3 bis 7 Kohlenstoffatomen, durch Halogen substituiertes Niederalkyl mit 1 bis etwa 6 Kohlenstoffatomen oder ein durch 1 bis 3 Phenylreste substituierter Kohlenwasserstoffrest mit 1 bis etwa 6 Kohlenstoffatomen oder ein durch 1 bis 3 Phenoxyreste substituierter Kohlenwasserstoffrest mit 1 bis etwa 6 Kohlenstoffatomen, wobei die Phenyl- und Phenoxyreste durch Halogen, Niederalkyl, Halogenalkyl, Halogenalkoxy oder Phenyl substituiert sein können, bedeutet,

zum Schutz gegen holzzerstörende und holzverfärbende Pilze besonders geeignet sind.

Die erfindungsgemäßen 1-Iod-1-alkinole und ihre Verwendung zum Schutz lebender Pflanzen ist Gegenstand der EP-A 0 066 761 und der EP-A 0 066 771.

Niederalkyl bedeutet erfindungsgemäß einen geradkettigen oder verzweigten Kohlenwasserstoffrest mit 1 bis etwa 6, bevorzugt mit 1 bis 4, Kohlenstoffatomen. Beispielsweise seien genannt: Methyl, Ethyl, Propyl, Isopropyl, Butyl, Isobutyl, Pentyl, Isopentyl, Hexyl und Isohexyl.

Cycloalkyl bedeutet erfindungsgemäß einen cyclischen Kohlenwasserstoffrest mit 3 bis 7, bevorzugt 5 oder 6, Kohlenstoffatomen. Beispielsweise seien genannt: Cyclopropyl, Cyclobutyl, Cyclopentyl, Cyclohexyl und Cycloheptyl.

Durch Halogen substituiertes Niederalkyl bedeutet erfindungsgemäß ein durch 1 bis 5, bevorzugt 1 bis 3, Fluor-, Chlor-, Brom- oder Iodatome substituierter geradkettiger oder verzweigter Kohlenwasserstoffrest mit 1 bis etwa 6, bevorzugt 1 bis 4, Kohlenstoffatomen. Beispielsweise seien genannt: Trifluormethyl, Trichlormethyl, Difluor-chlormethyl, Fluor-dichlormethyl.

Der durch 1 bis 3, bevorzugt einen, Phenylreste substituierte Kohlenwasserstoffrest enthält 1 bis 6, bevorzugt 1 bis 4, Kohlenstoffatome. Beispielsweise seien genannt: Benzyl, 4-Chlorbenzyl, 2,4-Dichlorbenzyl, 3,4-Dichlorbenzyl, 2,6-Dichlorbenzyl, 4-Fluorbenzyl, 4-Methoxybenzyl, 4-Methylbenzyl, 2-Chlor-4-methylbenzyl.

Der durch 1 bis 3, bevorzugt einen, Phenoxyrest substituierte Kohlenwasserstoffrest enthält 1 bis 6, bevorzugt 1 bis 4, Kohlenstoffatome. Beispielsweise seien genannt: Phenoxymethyl, 4-Chlorphenoxymethyl, 3,4-Dichlorphenoxymethyl, 2,4-Dichlorphenoxymethyl, 4-Fluorphenoxymethyl, 2,6-Dichlorphenoxymethyl.

Die Phenyl- und Phenoxyreste können gegebenenfalls substituiert sein. Beispielsweise seien genannt: Fluor, Chlor, Brom, Methyl, Ethyl, Propyl, Isopropyl, Methyl oder Ethyl, das durch 1 bis 3 Halogenatome, bevorzugt Fluor oder Chlor, substituiert ist, insbesondere Trifluormethyl, Trichlormethyl, Difluor-chlormethyl, FLuor-dichlormethyl, ω-Trifluormethyl und ω-Trichlormethyl, Methoxy oder Ethoxy, das durch 1 bis 3 Halogenatome, bevorzugt Fluor oder Chlor, substituiert ist, insbesondere bevorzugt Trifluormethoxy und Trichlormethoxy.

Im allgemeinen können die Phenyl- und Phenoxyreste durch 1 bis 3 weitere Reste substituiert sein.

Im besonderen wird die Verwendung der erfindungsgemäßen mikrobioziden Mittel der Formel

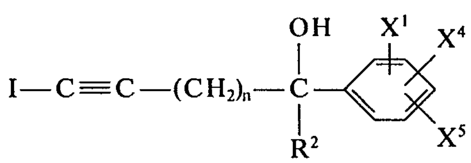

(II)

in der

$X^1$ und n die obengenannte Bedeutung haben,
$X^4$ Wasserstoff, Fluor oder Chlor bedeutet,
$X^5$ Wasserstoff oder Chlor bedeutet,
$R^2$ für Methyl, Ethyl, Isopropyl, tert.-Butyl oder Cyclohexyl steht,

zum Schutz technischer Materialien, gegen holzzerstörende und holzfärbende Pilze bevorzugt.

Die erfindungsgemäßen 1-Iod-1-alkinole können hergestellt werden, indem man die entsprechenden 1-Alkinole mit Iod in Gegenwart einer wäßrigen Alkalihydroxydlösung und gegebenenfalls in Gegenwart eines Verdünnungsmittels versetzt.

Die Einarbeitung der erfindungsgemäßen Wirkstoffe in Materialien, die Angriffen durch holzzerstörende und holzverfärbende Pilze ausgesetzt sind, inhibiert das Wachstum der Pilze; somit bleibt der ursprüngliche Wert der Materialien erhalten. Die Verbindungen sind nicht flüchtig, wasserunlöslich, lichtunempfindlich und thermostabil, so daß sie in solchen Materialien über längere Zeiträume beständig sind.

Technische Materialien im Rahmen der vorliegenden Erfindung sind Produkte, die selbst nicht in der Natur vorkommen, sondern aus natürlichen oder synthetischen Ausgangsmaterialien gefertigt werden. Die zu schützenden Produkte im Rahmen der vorliegenden Erfindung sind technische Materialien, die durch holzzerstörende und holzverfärbende Pilze zersetzt werden können.

Technische Materialien, die durch die erfindungsgemäßen Substanzen vor einer mikrobiellen Veränderung und Zerstörung durch holzzerstörende und holzverfärbende Pilze geschützt werden sollen, sind beispielsweise Papiere, Kartone und Holz.

Es seien beispielsweise Pilze der folgenden Gattungen genannt:

Chaetomium, wie Chaetomium globosum,
Coniophora, wie Coniophora cerebella,
Lentinus, wie Lentinus tigrinus,
Polyporus, wie Polyporus versicolor,
Pullularia, wie Pullularia pullulans,
Sclerophoma, wie Sclerophoma pityophila,
Trichoderma, wie Trichoderma viride.

Je nach ihrem Anwendungsgebiet können die erfindungsgemäßen Substanzen in die üblichen Formulierungen überführt werden, wie Lösungen, Emulsionen, Suspensionen, Pulver, Pasten und Granulate. Diese können in an sich bekannter Weise hergestellt werden, z. B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Benutzung von Verstreckmitteln, gegebenenfalls organische Solventien, als Hilfslösungsmittel verwendet werden können.

Organische Solventen für die Wirkstoffe können beispielsweise Alkohole, wie niedere Alkohole, vorzugsweise Ethanol oder Isopropanol, oder Benzylalkohol, Ketone wie Aceton oder Methylethylketon, flüssige Kohlenwasserstoffe, wie Benzinfraktionen, chlorierte Kohlenwasserstoffe, wie 1,2-Dichlorethan, sein.

Die erfindungsgemäßen mikrobioziden Mittel enthalten im allgemeinen 10 bis 100 Gew.-%, bevorzugt 50 bis 80 Gew.-%, der 1-Iod-1-alkinole als Wirkstoff.

Die Anwendungskonzentration der erfindungsgemäßen Substanzen richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 0,5 Gew.-%, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt: Benzimidazolyl-methylcarbamat, Tetramethyl-thiuramdisulfid, N-Fluordichlormethylthio-phthalimid und N,N-Dimethyl-N'-phenyl-(N'-fluordichlormethylthio)-sulfamid, Formaldehyd-abspaltende Verbindungen, wie Hemiformale, Oxazolidine, Hexahydro-s-triazine, N-Methylolamide und Phenolderivate, wie p-Chlorm-kresol, 2-Phenyl-phenol, (2,2'-Dihydroxy-5,5'-dichlor)-diphenylmethan.

## Herstellungsbeispiele

### Beispiel 1

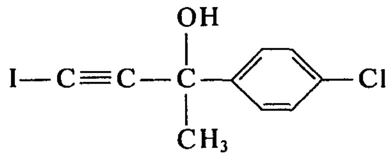

In eine Lösung von 180,6 g (1 Mol) 3-(4-Chlorphenyl)-1-butin-3-ol in 2000 ml Methanol werden bei 10 bis 15°C portionsweise 266,5 g (1,05 Mol) Iod eingetragen und dabei gleichzeitig 400 ml konzentrierte Natronlauge zugetropft. Nach 3 Stunden wird das Reaktionsgemisch in 2 l Wasser eingerührt und dabei das ausgeschiedene Öl in 500 ml Essigester aufgenommen. Die Essigester-Phase wird dreimal mit je 50 ml Wasser gewaschen, über Wasserfreiem Natriumsulfat getrocknet und anschließend unter vermindertem Druck eingedampft. Das zurückbleibende Öl kristallisiert beim Anreiben mit wenig

Petrolether. Man erhält so 230 g (75% der Theorie) 1-Iod-3-(4-chlorphenyl)-1-butin-3-ol als farblose Kristalle vom Schmelzpunkt 84 bis 85°C.

Beispiel 2

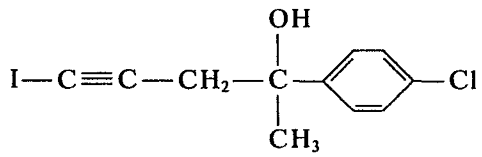

Zu einer Lösung von 58,4 g (0,3 Mol) 4-(4-Chlorphenyl)-1-pentin-4-ol in 600 ml Methanol werden bei 20 bis 25°C unter leichter Außenkühlung 144 ml konzentrierte Natronlauge zugetropft und bei gleichzeitig 91,5 g (0,36 Mol) Iod portionsweise eingetragen. Nach 8 Stunden wird das Reaktionsgemisch in 1,5 ml Wasser eingerührt. Man extrahiert zweimal mit je 250 ml Essigester, wäscht die organische Phase mit Wasser und destilliert das Lösungsmittel unter vermindertem Druck ab. Man erhält so 85 g (88,4% der Theorie) 1-Iod-4-(4-chlorphenyl)-1-pentin-4-ol als schwach gelbliche gefärbtes Öl vom Brechungsindex $n_D^{20}$: 1,6078.

In entsprechender Weise werden die nachfolgenden Beispiele der allgemeinen Formel (I)

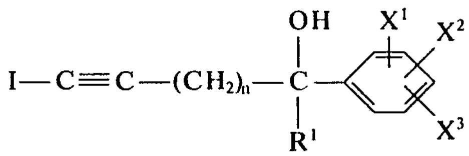

(I)

erhalten.

| Beispiel Nr. | n | $X^1$ | $X^2$ | $X^3$ | R | Physikal. Konstante (Schmelzpunkt °C: Brechungsindex $n_D^{20}$) |
|---|---|---|---|---|---|---|
| 3 | 0 | 4-Cl | 3-Cl | H | $CH_3$ | 72 bis 74 |
| 4 | 0 | H | H | H | $CH_3$ | 1,5661 |
| 5 | 0 | 4-Cl | H | H | $C_3H_7$-iso | 1,593 |
| 6 | 0 | 4-F | H | H | $C_3H_7$-iso | 57 bis 59 |
| 7 | 1 | 4-Cl | 3-Cl | H | $CH_3$ | 1,6107 |
| 8 | 1 | 4-F | H | H | $CH_3$ | 1,5781 |
| 9 | 1 | 4-Br | H | H | $CH_3$ | 1,622 |
| 10 | 1 | H | H | H | $C_3H_7$-iso | 1,5839 |
| 11 | 1 | 4-Cl | H | H | $C_3H_7$-iso | 1,5878 |
| 12 | 1 | 4-Cl | H | H | $C_2H_5$ | 1,5752 |
| 13 | 1 | 4-F | H | H | $C_3H_7$-iso | 1,5668 |
| 14 | 1 | 4-Cl | 2-Cl | H | $C_2H_5$ | 1,6039 |
| 15 | 1 | 4-Cl | 2-Cl | H | $CH_3$ | 1,6148 |
| 16 | 1 | 4-F | H | H | 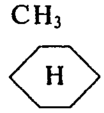 | 1,5771 |
| 17 | 1 | H | H | H | 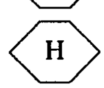 | 1,5912 |

Fortsetzung

| Beispiel Nr. | n | $X^1$ | $X^2$ | $X^3$ | R | Physikal. Konstante (Schmelzpunkt °C: Brechungsindex $n_D^{20}$) |
|---|---|---|---|---|---|---|
| 18 | 1 | 4-F | H | H | $C_2H_5$ | 1,5702 |
| 19 | 1 | H | H | H | $C_2H_5$ | 1,5908 |
| 20 | 1 | 4-Cl | 3-Cl | H | $C_2H_5$ | 1,6060 |
| 21 | 1 | H | H | H | $CH_3$ | 1,6017 |

## Anwendungsbeispiele

### Beispiel 22

### Wirkung gegen Pilze

In einen Agar, der aus Bierwürze und Pepton hergestellt wurde, wurden die erfindungsgemäßen Verbindungen in abgestuften Konzentrationen zwischen 1 und 5000 mg/l Versuchsprobe eingearbeitet. Nach dem Erstarren des Agars erfolgte die Kontamination der so hergestellten Agarproben mit Reinkulturen verschiedener Testpilze (s. Tabelle).

Nach zweiwöchiger Lagerung bei 28°Cund 60 bis 70%relativer Luftfeuchtigkeit wurde ausgewertet. In der Tabelle ist als minimale Hemmkonzentration (MHK) die geringste in einer Agarprobe enthaltene Konzentration der Substanz angegeben, bei der keinerlei Bewuchs durch die verwendete Art erfolgte.

In diesem Test zeigen z. B. folgende Verbindungen eine sehr gute Wirkung:

Verbindungen gemäß den Herstellungsbeispielen 1 und 2.

Tabelle 1
Wirkstoff gegen Pilze

| Wirkstoff | MHK-Wert in mg/l für folgende Spezies | | | | | | |
|---|---|---|---|---|---|---|---|
| | Coniophora cerebella | Lentius tigrinus | Pullularia pullulans | Sclerophoma pityophila | Polyporus versicolor | Chaetonium globosum | Trichoderma viride |
| 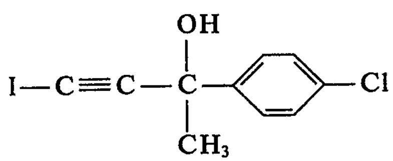 (1) | 1,0 | 5,0 | 2,0 | 0,1 | 2,0 | 35,0 | 20,0 |
| 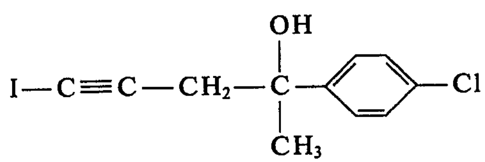 (2) | | | 5,0 | | | 75,0 | 100,0 |

## Patentansprüche

1. Verwendung von 1-Iod-1-alkinolen der Formel

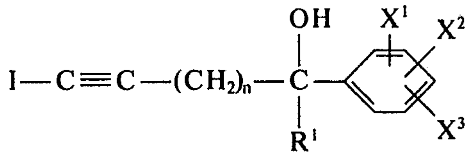

in der
$X^1$, $X^2$ und $X^3$ gleich oder verschieden sind und Wasserstoff, Fluor, Chlor oder Brom bedeuten,
n für die Zahlen 0 oder 1 steht und
$R^1$ Niederalkyl mit 1 bis etwa 6 Kohlenstoffatomen, Cycloalkyl mit 3 bis 7 Kohlenstoffatomen, durch Halogen substituiertes Niederalkyl mit 1 bis etwa 6 Kohlenstoffatomen oder ein durch 1 bis 3 Phenylreste substituierter Kohlenwasserstoffrest mit 1 bis etwa 6 Kohlenstoffatomen oder ein durch 1 bis 3 Phenoxyreste substituierter Kohlenwasserstoffrest mit 1 bis etwa 6 Kohlenstoffatomen, wobei die Phenyl- und Phenoxyreste durch Halogen, Niederalkyl, Halogenalkyl, Halogenalkoxy oder Phenyl substituiert sein können, bedeutet,
zum Schutz gegen holzzerstörende und holzverfärbende Pilze.

2. Verwendung von 1-Iod-1-alkinolen nach Anspruch 1, in Anwendungskonzentrationen im Bereich von 0,001 bis 5 Gew.-% bezogen auf das zu schützende Material.

## Claims

1. Use of 1-iodo-1-alkynols of the formula

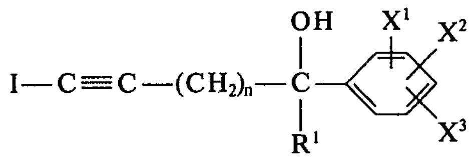

in which
$X^1$, $X^2$ and $X^3$ are identical or different and denote hydrogen, fluorine, chlorine or bromine,
n represents the numbers 0 or 1 and
$R^1$ denotes lower alkyl with 1 to about 6 carbon atoms, cycloalkyl with 3 to 7 carbon atoms, halogen-substituted lower alkyl with 1 to about 6 carbon atoms or a hydrocarbon radical which has 1 to about 6 carbon atoms and is substituted by 1 to 3 phenyl radicals or a hydrocarbon radical which has 1 to about 6 carbon atoms and is substituted by 1 to 3 phenoxy radicals, it being possible for the phenyl and phenoxy radicals to be substituted by halogen, lower alkyl, halogenalkyl, halogenalkoxy or phenyl,
for protection against fungi which destroy or discolour wood.

2. Use of 1-iodo-1-alkynols according to Claim 1 in use concentrations in the range of 0.001 to 5% by weight, based on the material to be protected.

## Revendications

1. Utilisation des 1-iodo-1-alcynols de formule

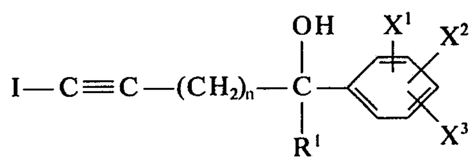

dans laquelle
$X^1$, $X^2$ et $X^3$, ayant des significations identiques ou différentes, représentent l'hydrogène, le fluor, le chlore ou le brome,
n est égal à 0 ou 1, et
$R^1$ représente un groupe alkyle inférieur en $C_1-C_6$ environ, cycloalkyle en $C_3-C_7$, alkyle inférieur en $C_1-C_6$ environ substitué par des halogènes, ou un reste hydrocarboné en $C_1-C_6$ environ substitué par 1 à 3 groupes phényle ou un reste hydrocarboné en $C_1-C_6$ environ substitué par 1 à 3 groupes phénoxy, les groupes phényle et phénoxy pouvant être substitués par des halogènes, des groupes alkyle inférieurs, halogénoalkyle, halogénoalcoxy ou phényle,
pour la protection contre les mycètes détruisant le bois et colorant le bois.

2. Utilisation des 1-iodo-1-alcynols selon la revendication 1, à des concentrations d'utilisation dans l'intervalle de 0,001 à 5% en poids par rapport au matériau à protéger.